Europäisches Patentamt

European Patent Office

Office européen des brevets

(19)

(11) Publication number: **0 171 243 B1**

## EUROPEAN PATENT SPECIFICATION

(12)

(45) Date of publication of patent specification: **09.09.92**

(51) Int. Cl.5: **G01N 33/574**, G01N 33/76, G01N 33/68, G01N 33/573, G01N 33/561

(21) Application number: **85305362.7**

(22) Date of filing: **26.07.85**

The file contains technical information submitted after the application was filed and not included in this specification

(54) **Method for diagnosing cancer.**

(30) Priority: **07.08.84 JP 165351/84**

(43) Date of publication of application:
**12.02.86 Bulletin 86/07**

(45) Publication of the grant of the patent:
**09.09.92 Bulletin 92/37**

(84) Designated Contracting States:
**CH DE FR GB IT LI SE**

(56) References cited:
**US-A- 4 455 380**

**CHEMICAL ABSTRACTS, vol. 96, no. 11, 15 March 1982, Columbus, OH (US); J.MIYAZAKI et al., p. 283, no. 82101c**

**CANCER, vol. 55, no. 1, 01 January 1985; T.ISHIGURO et al., pp. 156-159**

**ELECTROPHOR. '83, ADV. METHODS, BIOCHEM. CLIN. APPL., PROC. INT. CONF., 1983 (published 1984), Walter de Gruyter & Co.; K.TAKETA et al., pp. 611-618**

**CHEMICAL ABSTRACTS, vol. 102, no. 9, 04 March 1985, Columbus, OH (US); T.ISHIGURO et al., p. 265, no. 75036z**

**CHEMICAL ABSTRACTS, vol. 101, no. 1, 02 July 1984, Columbus, OH (US); K.TAKETA et al., p. 420, no. 4856k**

**CHEMICAL ABSTRACTS, vol. 95, no. 15, 12 October 1981, Columbus, OH (US); J.BREBOROWICZ et al., p. 291, no. 128616j**

(73) Proprietor: **Kyowa Medex Co. Ltd.**
**6-1 Ohtemachi Itchome Chiyoda-ku Tokyo(JP)**

(72) Inventor: **Ishiguro, Tatsuya**
**57, Shimogamominakuchi-machi Sakyo-ky Kyoto-shi Kyoto-fu(JP)**

(74) Representative: **Lambert, Hugh Richmond et al**
**D. YOUNG & CO. 10 Staple Inn London, WC1V 7RD(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

**Description**

This invention relates to a method for the differential diagnosis of primary and metastatic cancers by qualitative and quantitative lectin-based immuno-serological assays of cancer-related antigens in body fluids. More particularly the invention relates to the differential diagnosis of primary and metastatic hepatic carcinoma, and the differential diagnosis of oophoroma and metastatic hepatic carcinoma by affinity cross-linked immuno-electrophoresis (ACIE) of $\alpha$-fetal protein (AFP) using selected lectin pairs.

Specific immuno-serological assays based on the immunogenicity of cancer-specific glycoproteins as tumor markers are already used in the diagnosis of cancer. Such markers include, for example $\alpha$-fetoprotein (hereinafter referred to as AFP), carcinoembryonic antigen (hereinafter referred to as CEA), $\gamma$-glutamyltranspeptidase (hereinafter referred to as $\gamma$-GTP) and human chorionic gonadotropin (hereinafter referred to as HCG).

For example, the measurement of serum AFP is used in the specific immunoserological diagnosis of primary hepatic carcinoma. However, if the increase of serum AFP is small, it is difficult to decide whether it is caused by the regeneration of hepatocytes or by hepatoma cells. This is for the reason that serum AFP increase not only in the case of primary hepatic carcinoma, but also sometimes in the case of metastasis to the liver resulting from oophoroma, and sometimes from gastric cancer and pancreatic cancer (metastatic hepatic carcinoma). Thus the differential diagnosis of primary hepatic cancer and oophoroma is hard to make on the basis of mere quantitative change in serum AFP.

Lectin-based affinity cross-linked immunoelectrophoretic assays of serum AFP are known, and have been suggested as a basis for the differential diagnosis of liver disease. For example, Kanzo 1981, 22(11), 1559-69 (Chem.Abs. 1982 Vol.96 No.11, 82101c) discloses the diagnosis of hepatocellular carcinoma and "metastatic" liver tumor by ACIE with concanavalin A (Con A) and lentil lectin (LCH).

It is reported that with Con A, AFP appears in two fractions, one reactive and one non-reactive, and in the case of hepatocellular carcinoma the AFP is mainly of the Con A reactive type, whereas for digestive tract tumors the AFP is 50% non-reactive.

In the case of LCH affinity, it is reported that three types of reactivity have been noted, namely a non-reactive fraction, a weakly reactive fraction and a strongly reactive fraction. However, it is also reported that, although AFP from cirrhotic liver is mainly LCH non-reactive, AFP produced by malignant tumors shows a wide distribution in affinity to LCH, with the LCH non-reactive fraction varying widely from 5 to 100%.

Thus, whilst the authors of that article do speculate that analysis of AFP by ACIE using Con A and LCH may prove useful in the differential diagnosis of liver disease, their results using LCH are somewhat inconclusive, and, in fact, show extreme variability. Thus, in Table I the LCH - affinities of AFP from hepatocellular carcinoma and gastrointestinal tumor are reported as 41.1± 34.3 and 31.6± 41.9 respectively.

In the diagnosis and treatment of liver disease, it is clearly important to be able to differentiate between primary and secondary (metastatic) disease, and in accordance with this invention, it has been found that this can be achieved using specifically selected pairs of lectins as the basis of analysis of serum AFP by affinity cross-linked immuno electrophoresis (ACIE).

More specifically, the present invention, in one aspect, relates to a method of differentiating between primary and metastatic hepatic carcinoma, which comprises subjecting a sample of body fluids, preferably a serum sample, from the patient to cross-immunoaffinoelectrophoresis (ACIE) using a pair of lectins selected from the following lectin pairs:

Con A : LCH

Con A : PHA-E

RCA -I : LCH

RCA -I : BSA-II

BSA -II : LCH

to obtain, for each lectin of the selected pair, an $\alpha$-fetoprotein (AFP) specific fraction binding pattern, and using the two patterns obtained to differentiate, as between primary and metastatic disease.

In a second and related aspect, the invention relates to a method of differentiating between oophoroma and metastatic hepatic carcinoma, which comprises subjecting a sample of body fluids, preferably a serum sample, from the patient to cross-immunoaffinoelectrophoresis (ACIE) using a pair of lectins selected from the following lectin pairs:

PHA -E : RCA-I

PHA -E : BSA-II

to obtain, for each lectin of the selected pair, an $\alpha$-fetoprotein (AFP) specific fraction binding pattern, and using the two patterns obtained to differentiate between oophoroma and metastatic hepatic carcinoma.

Preferably, in the above methods, peroxidase (POD)-labelled protein A, 4-methoxy-1-naphthol and

EP 0 171 243 B1

hydrogen peroxide are used in the development of the said binding patterns in the ACIE.

Suitable body fluid samples for use in the present invention include, for example, blood, urine, saliva, gastric juice, intestinal juice, pancreatic juice, bile, cell tissue fluid, edema fluid, ascitic fluid, hydrothorax fluid, hydropericardic fluid, synovial fluid, mother's milk, nasal mucus, phlegm, tear, sweat, etc., and it is particularly preferable to use blood in the form of serum or plasma.

The lectins to be used in the present invention include jack bean lectin (hereinafter referred to as Con A) and lentil lectin (hereinafter referred to as LCH) which are lectins capable of being specifically bound to $\alpha$-D-glucose and $\alpha$-D-mannose; isolectin PHA-E of kidney bean lectin (hereinafter referred to as PHA) which is a lectin capable of being specifically bound to N-acetylgalactosamine; isolectin RCA-I of castor bean lectin (hereinafter referred to as RCA) which is a lectin capable of being specifically bound to $\beta$-D-galactose; and isolectin BSA-II of banderilla bean lectin (hereinafter referred to as BSA) which is a lectin capable of being specifically bound to N-acetylglucosamine. In the present invention the ACIE method is used for separation and identification of the cancer-related glycoantigen viz: the AFP. That is, a sample containing a cancer-related glycoantigen is subjected to one-dimensional electrophoresis separation in a lectin-containing gel, and then the individual separated fractions are subjected to two-dimensional electrophoresis and coloring in an antiserum-containing gel, whereby the individual fractions can be quantitatively determined as sharp precipitate peaks. The lectin bindability then can be determined from the specific change in the migration distance.

In the present invention, an enzyme immunoassay is used for developing the specific fraction binding pattern after the ACIE. Particularly preferred is a coloring system comprising POD-labelled protein A, 4-methoxy-1-naphthol and hydrogen peroxide which gives a clear, rapid and simple means of developing the pattern with a high sensitivity and a high separability.

As already indicated by the prior art the differential diagnosis of cancer may be possible to some extent by a single bindability to lectin, but not always very satisfactorily, and it is the basis of the present invention that the diagnosis can be made more definitively by a combination of at least two lectin affinities and in particular, in the pairs already mentioned.

More particularly, and as indicated above it is known that AFP which is a glycoprotein existing in the blood of patients suffering from primary hepatic carcinoma, gonadal teratoma, and oophoroma, can be separated by ACIE into several fractions due to the difference in bindability to various lectins. For example, with Con A, AFP is separated into two fractions, i.e. a Con A-bound fraction and a Con A-unbound fraction. It is ontogeneologically known that the Con A-bound AFP originates from the hepatocytes and the Con A-unbound AFP originates mostly from the vitellicle. When the serum AFP of patients suffering from primary hepatic carcinoma is assayed in the present invention, a fraction originating partly from the vitellicle (hereinafter referred to as peak b) is found and also an AFP fraction originating mostly from the embryonic hepatocytes (hereinafter referred to as peak a). However, in metastatic hepatic carcinoma, only the AFP fraction originating from the vitellicle (peak b) is found. With LCH, AFP is likewise separated into 4 fractions giving four peaks A, B, C and D. It can be established from changes in AFP-producing sites with the elapse of time, that peaks A and C are fractions originating from the hepatocytes, and peaks B and D are fractions originating from the vitellicle. Thus it can be seen that in AFP fraction patterns of primary hepatic carcinoma only peaks A and C are found whereas in metastatic hepatic carcinoma, peak B occurs as well as peaks A and C.

That is the AFP fractions produced in the embryonic hepatocytes mainly appear in the case of the primary hepatic carcinoma, whereas in the case of metastatic hepatic carcinoma, a fraction produced in the vitellicle is found as well as the AFP originating from fetal liver. Thus, by this method and by comparing the specific binding patterns obtained with Con A and LCH it is possible to make a differential diagnosis as between primary and metastatic hepatic carcinoma.

The amounts of sample and of the two lectins used in the assay and the amounts and proportions of POD-labelled protein A and enzyme substrate used in the development of the binding pattern and the various reaction conditions, e.g. time and temperature, depend on the substance to be assayed, the particular lectin used and the kind of carrier, used in the electrophoresis, and thus the most appropriate conditions must be determined experimentally for each assay.

The method of the present invention is described in more detail below.

In general, in carrying out the electrophoresis a sample of body fluid, preferably 5 to 10ml, is first subjected to one-dimensional electrophoresis in a lectin-containing gel containing one or other of the selected pair of lectins.

After the one-dimensional electrophoresis, each of the separated fractions is subjected to a second electrophoresis in a second direction in an antiserum-containing gel following which the specific binding pattern is developed using an enzyme immunoassay.

3

The concentration of lectin in the first gel depends on the lectin used, and generally the separability of the AFP fraction depends also on that concentration. A feature of the present invention is that the selected lectins offer a highly sensitive assay operable even at a low lectin concentrations of, for example, from 200 to 600 $\mu$g/ml.

A variety of gels can be used as the carrier, although in the case of two-dimensional ACIE, it is preferable to use an agarose gel because of its low gel deformation. The carrier gel can be prepared by conventional procedures. For example, a predetermined amount of agarose is added to distilled water, or to a suitable dilute buffer solution, for example a barbital buffer solution at a pH of about 8.6 or a Tris-hydrochloric acid buffer solution, and heated at a temperature of from 60 to 80°C with gentle stirring to dissolve the agarose. The solution is then spread on a flat plate and left to cool. The gel concentration is usually 0.5 to 2.0% by weight, preferably 0.8 to 1.0% by weight. An antiseptic can be added to the gel, if needed.

Preferably the electrophoresis is carried at a temperature of not higher than 15°C, preferably 5 to 10°C in order to avoid drying out and deformation of the gel at these lower temperatures.

The greater the one-dimensional migration distance of ACIE, the greater the separation of the AFP fractions. However, a corresponding large amount of lectin will be required as well as a long migration time. In practice 6 cm is a sufficient migration distance. As a control in the electrophoresis, a coloured substance, e.g. Bromophenol Blue (hereinafter referred to as BPB) - bound albumin is diffused in parallel with the assay sample.

Overall, the two-dimensional electrophoresis can be completed within 2 to 6 hours, preferably 2 to 4 hours.

The invention is illustrated by the following:

Example 1

Preliminary differential diagnosis of hepatic carcinoma using LCH made by Pharmacia Fine Chemicals Inc., Sweden) -ACIE:

(a) Preparation of assay samples:

Individual serum samples from 48 patients with primary hepatic carcinoma and 8 patients with metastatic hepatic carcinoma were diluted with normal human serum (AFP: less than 5 ng/ml) to give samples with an adjusted AFP concentration of less than 30,000 ng/ml.

(b) Preparation of LCH-containing agarose plate (for one-dimensional electrophoresis):

1.0% agarose (made by FMC Marine Colloids Division, U.S.A.) was dissolved in a barbital buffer solution having pH 8.6 and an ionic strength of 0.5 followed by the addition of 200 $\mu$g/ml LCH. 15ml of the LCH-containing agarose gel was then poured into a 110 x 125mm gel mould (FMC Marine Colloids Division) and coagulated to produce an LCH-containing agarose plate having a thickness of about 1mm.

(c) Preparation of anti-AFP serum-containing agarose plate (for two-dimensional electrophoresis):

To 10 ml. of a 1.0% agarose solution were added 20 $\mu$l of anti-AFP serum (DAKO Co., Denmark). 10 ml of the anti-AFP serum-containing agarose gel were then poured into the same gel mould and coagulated to make an anti-AFP serum-containing agarose plate having a thickness of about 1mm.

(d) Preparation of diluted POD-labelled protein A solution

20$\mu$l of POD-labelled protein A (E.Y. Laboratories, U.S.A.) were added to 10ml of a dilute buffer solution prepared by dissolving 14.0g of NaCl in 1l of barbital buffer having pH 8.6, to make a 0.2% dilute solution of POD-labelled protein A.

(e) Preparation of coloring reagent solution:

200 $\mu$l of 4-methoxy-1-naphthol (Aldrich Co., U.S.A.), 10ml of 0.05M Tris buffer solution (12.113 g/l trishydroxymethylaminomethane and 0.1M HCl in a ratio of 100:77, pH 7.6) and a drop of 30% $H_2O_2$ were mixed together to make a coloring reagent solution immediately before use.

(f) Measurement of AFP:

10 $\mu$l serum samples prepared as in section (a) were poured into 4mm holes in the LCH-containing agarose plate prepared as in section (b) and then subjected to one-dimensional electrophoresis. Electrophoresis was carried out using a barbital buffer solution (pH 8.6) at 3 mA/cm for 90 minutes at 10°C.

As a control, human serum albumin (fraction V, made by Sigma Co., U.S.A., containing 5$\mu$l of 0.01% BPB) was used and the migration distance marked (about 6cms.)

After the one-dimensional electrophoresis, the agarose plate was cut into 1 cm. wide strips parallel to the direction of migration and placed on top of the anti-AFP serum-containing agarose plate prepared in section (c) and in close contact with the anti-AFP serum-containing gel. The assembly was then subjected to a second electrophoresis in the direction perpendicular to the direction of the original one-dimension development, at 2.5 mA/cm for 3 hours at 10°C.

After the migration, the plate was treated with an adsorbent pad (Nihon Shoji Co., Japan) for 2 to 3 minutes for elimination of any unbound substances, and then washed with a phosphate buffer solution (pH 7.2) for 30 minutes. The plate was then dried with an adsorbent pad, and 10 ml of POD-labelled protein A, prepared as in section (d), was poured onto the flat plate. After reaction for 30 minutes, the plate was dried with an adsorbent pad, washed with a phosphate buffer solution (pH 7.2) for 30 minutes and redried with an adsorbent pad. 10ml of the coloring reagent solution, prepared as in section (e), were then added and the plate allowed to react for 2 to 3 minutes. The developed plate was then washed with water for 2 to 3 minutes and dried with an adsorbent pad. The distance from the albumin position obtained by the one-dimensional electrophoresis to the migration peak obtained in the two-dimensional electrophoresis was measured.

From these results, typical migration patterns of sera from patients suffering from hepatic carcinoma can be obtained and are seen to be divided into four types: Type I in which only peak A appears; Type II in which only peak C appears; Type III in which two peaks, i.e. both peak A and peak C appear; and Type IV in which peak B appears with or without peaks A and/or peak C.

Type III can be further sub-divided quantitatively into two sub-types: Type IIIa in which peak A is higher than peak C and Type IIIb in which peak C is higher than peak A.

Type IV can be further sub-divided into three sub-types: Type IVa in which two peaks, i.e. peak A and peak B, appear; Type IVb in which peaks A, B and C appear; and Type IVc in which only peak B appears.

As shown in Table 1, AFP in the sera from patients suffering primary hepatic carcinoma is fractionated into Type I, Type II and Type III, whereas that from patients with metastatic hepatic carcinoma is all fractionated into Type IV, thus enabling a preliminary differentiation between the primary hepatic carcinoma and metastatic hepatic carcinoma.

## Table 1

### AFP fraction appearance frequency by LCH-ACIE

| Fraction pattern | Fraction peaks | Primary hepatic carcinoma | Metastatic hepatic carcinoma | |
|---|---|---|---|---|
| Type I | A | 2 (4.2%) | 0 | |
| Type II | C | 6 (12.5%) | 0 | |
| Type III | | | | |
| Type IIIa | A >C | 21 (43.7%) | 0 | |
| Type IIIb | A <C | 19 (39.6%) | 0 | |
| Type IV | | | | |
| Type IVa | A B | 0 | 4 | (50.0%) |
| Type IVb | A B C | 0 | 2 | (25.0%) |
| Type IVc | B | 0 | 2 | (25.0%) |
| | | 48 | 8 | |

Example 2

Preliminary differential diagnosis of hepatic carcinoma by Con A-ACIE

Exactly the same procedure was carried out as in Example 1, except that a Con A (Pharmacia Fine Chemicals)-containing agarose plate (Con A: 600 $\mu$g/ml) was used in place of the LCH-containing agarose plate.

From these results a second set of typical migration patterns of sera of patients suffering from hepatic carcinoma by Con A-ACIE can be obtained and are seen to be divided into three types: Type 1 in which only peak a appears; Type 2 in which peak a and peak b both appear; and Type 3 in which only peak b appears.

As shown in Tables 2 and 3, AFP in the sera from patients with primary hepatic carcinoma is fractionated into Type 1 (only peak a) and Type 2 (peak a > peak b), whereas that from patients with metastatic hepatic carcinoma is fractionated into Type 2 (peak a < peak b) and Type 3 thus enabling a preliminary differentiation between primary and metastatic hepatic carcinoma.

Table 2

| AFP fraction appearance frequency by Con A-ACIE | | | |
|---|---|---|---|
| Fraction pattern | Fraction peaks | Primary hepatic carcinoma | Metastatic hepatic carcinoma |
| Type 1 | a | 30 (62.5%) | 0 |
| Type 2 | a b | 18*(37.5%) | 6 (75%) |
| Type 3 | b | 0 | 2 (25%) |
| | | 48 | 8 |

* At least two measurements were made for each case, and every case where Type 2 appeared in at least one measurement was classified into Type 2.

## Table 3

### Height and width of peak b by Con A-ACIE

| | Primary hepatic carcinoma | Metastatic hepatic carcinoma |
|---|---|---|
| Number of investigations | 18 | 6 |
| Average height of peak b*3 | $14.1 \pm 3.6$*[1] | $44.5 \pm 20.2$*[2] |
| Width of peak b in mm | 8 - 12 | 17 - 68 |

*1    The higher value was adopted where several measurements were made for each case.

*2    The lower value was adopted where several measurements were made for each case.

*3    Average height of peak b as expressed by the formula

$$\frac{\text{Height of peak b}}{\text{Height of peak a + height of peak b}} \times 100\%$$

Example 3

Preliminary differential diagnosis of oophoroma and primary and metastatic hepatic carcinoma by PHA-E-ACIE:

Following the same procedure as in Example I ACIE assays were carried out on individual serum samples from 5 patients with primary hepatic carcinoma, 4 patients with metastatic hepatic carcinoma, 4 patients with oophoroma, and one patient with orchioncus except that a PHA-E (E.Y. Laboratories, USA) - containing agarose plate (PHA-E: 200 $\mu$g/ml) was used in place of the LCH-containing agarose plate. Four migration peaks were identified on the basis of the migration distances shown in Table 4, and identified herein as peaks A, B, C and D.

7

Table 4

| Classification of individual peaks by migration distance in mm. | | | |
|---|---|---|---|
| Peak A | Peak B | Peak C | Peak D |
| 21.0< | 16.0 - 21.0 | 12.0 - 16.0 | 12.0> |

From these results, the migration patterns of sera of patients suffering from oophoroma, primary and metastatic hepatic carcinoma and orchioncus are established and shown to be divided into two types: Type 1 in which only peaks C and D appear either singly or together; and Type 2 in which peaks A and C or B and C appear together; peak B appears alone; or peaks B, C and D all appear. Type 2 is thus characterized by appearance of either peak A or peak B (shown as underlined in Table 5).

No specific change in the AFP fractions appearing in amniotic fluid during pregnancy period was observed using PHA-E-ACIE. However, distinctive migration patterns were observed with the sera of cancer patients, as shown in Table 5.

Table 5

| AFP fraction appearance frequency by PHA-E-ACIE | | | | | |
|---|---|---|---|---|---|
| Fraction pattern | Fraction peaks | Primary hepatic carcinoma | Metastatic hepatic carcinoma | Oophoroma | Orchioncus |
| Type 1 Type 2 | C, D, CD AC, B, BC, BCD | 5 0 | 0 4 | 1 3 | 0 1 |
| | | 5 | 4 | 4 | 1 |

Example 4

Preliminary differential diagnosis of oophoroma and primary and metastatic hepatic carcinoma by RCA-I-ACIE:

Using the same serum samples as in Example 3 RCA-I ACIE assays were carried out in the same manner as in Example 1, except that an RCA-I (E.Y. Laboratories) - containing agarose plate (RCA-I: 200$\mu$g/ml) was used in place of the LCH-containing agarose plate.

Four migration peaks were identified on the basis of the migration distances shown in Table 4 and identified herein as peaks A, B, C and D.

From these results, the migration patterns of sera from patients suffering from oophoroma, primary and metastatic hepatic carcinoma and orchioncus were established and shown to be divided into three types. Type 1 in which peaks C and D appear either singly or together; Type 2 in which peaks A and C or A and D appear, or in which all three peaks A, C and D appear together; and Type 3 in which either peaks A, B and D appear together, or just peaks B and D appear. Type 2 and Type 3 are thus characterized by appearance of peak A and/or B (shown as underlined in Table 6).

No specific change in the AFP fractions appearing in amniotic fluid during pregnancy were observed using RCA-I-ACIE. However, distinctive migration patterns were observed in the sera of cancer patients as shown in Table 6.

Table 6

| AFP fraction appearance frequency by RCA-I-ACIE | | | | | |
|---|---|---|---|---|---|
| Fraction pattern | Fraction peaks | Primary hepatic carcinoma | Metastatic hepatic carcinoma | Oophoroma | Orchioncus |
| Type 1<br>Type 2<br>Type 3 | C, D, CD<br>AC, AD, ACD<br>ABD, BD | 5<br>0<br>0 | 1<br>2<br>1 | 0<br>3<br>1 | 0<br>1<br>0 |
| | | 5 | 4 | 4 | 1 |

Example 5

Preliminary differential diagnosis of oophoroma and primary and metastatic hepatic carcinoma by BSA-II-ACIE:

Using the same serum samples as in Example 3 BSA-II ACIE assays were carried out in the same manner as in Example 1 except that a BSA-II (E.Y. Laboratories) - containing agarose plate (BSA-II: 200 $\mu$g/ml) was used in place of the LCH-containing agarose plate.

Four migration peaks were identified on the basis of the migration distances shown in Table 4, and identified herein as peaks A,B, C and D.

From these results the migration patterns of sera from patients suffering from oophoroma, primary and metastatic hepatic carcinoma and orchioncus are established and shown to be divided into two types: Type 1 in which only peak C or D appear as single peaks; and Type 2 in which peaks A, B and D all appear or just peaks B and D. Type 2 is thus characterized by appearance of peak A and/or peak B (shown as underlined in Table 7).

No specific change in the AFP fractions appearing in amniotic fluid during pregnancy period were observed in BSA-II-ACIE. However, distinctive migration patterns were observed in the sera of cancer patients, as shown in Table 7.

Table 7

| AFP fraction appearance frequency by BSA-II-ACIE | | | | | |
|---|---|---|---|---|---|
| Fraction pattern | Fraction peaks | Primary hepatic carcinoma | Metastatic hepatic carcinoma | Oophoroma | Orchioncus |
| Type 1<br>Type 2 | C, D<br>ABD, BD | 5<br>0 | 1<br>3 | 4<br>0 | 1<br>0 |
| | | 5 | 4 | 4 | 1 |

The following Examples illustrate the specific methods of the present invention in confirming the differential diagnosis of primary and metastatic hepatic carcinoma, on the one hand, or oophoroma and metastatic hepatic carcinoma on the other.

Example 6

Final differential diagnosis of primary and metastatic hepatic carcinoma by combination of Con A and LCH:

The cases of obscure differentiation in Example 2, that is, the four cases showing a relatively high peak b value (14% or higher) in the primary hepatic carcinoma and the six cases showing a relatively low peak b value (45% or lower) in the metastatic hepatic cancer in the patient sera classified into type 2 by Con A were checked by the results of fraction patterns by LCH (Example 1).

As a result, it was found that all the primary hepatic carcinoma belonged to type III, and that all the metastatic hepatic carcinoma belonged to Type IV, thus enabling a clearly established differentiation as between primary and metastatic disease.

Example 7

Final differential diagnosis of primary and metastatic hepatic carcinoma by combination of Con A and PHA-E:

The cases of obscure differentiation in Example 2, that is, the cases showing a relatively high peak b value (14% or higher) in the primary hepatic cancer and the cases showing a relatively low peak b value (45% or lower) in the metastatic hepatic carcinoma in the patient sera belonging to type 2 by Con A were compared with the results of Example 3.

As a result, it was confirmed that all the primary hepatic carcinoma belonged to Type 1, and that all the metastatic hepatic carcinoma belonged to Type 2.

Example 8

Final differential diagnosis of oophoroma and metastatic hepatic carcinoma by combination of PHA-E and RCA-I:

The cases of obscure differentiation in Example 3, that is, the sera of patients suffering from oophoroma showing Type 1 by PHA-E ACIE were classified into Type 3 by comparison with the results of the RCA-I ACIE (Example 4).

Conversely the sera of patients suffering from the metastatic hepatic carcinoma showing Type 1 by RCA-I ACIE (Example 4) were classified into Type 2 by comparison with the results of the PHA-E ACIE (Example 3).

Example 9

Final differential diagnosis of oophoroma and metastatic hepatic carcinoma by combination of PHA-E and BSA-II.

The cases of obscure differentiation in Example 3 that is, sera of patients suffering from oophoroma showing Type 1 by PHA-E ACIE were classified into Type 1 by comparison with the results of the BSA-II ACIE (Example 5). Conversely the sera of patients suffering from the metastatic hepatic carcinoma showing Type 1 by BSA-II ACIE (Example 5) were classified into Type 2 by comparison with the results of the PHA-E ACIE (Example 3).

Example 10

Final differential diagnosis of cancer by combination of RCA-I and LCH

The cases of obscure differentiation in Example 4, that is, sera of patient suffering from metastatic hepatic carcinoma showing Type 1 by RCA-I ACIE were classified into Type IVb by comparison with the results of LCH-ACIE (Example 2).

Example 11

Final differential diagnosis of cancer by combination of RCA-I and BSA-II:

The cases of obscure differentiation in Example 4 that is, sera of patients suffering from metastatic hepatic cancer showing Type 1 by RCA-I ACIE were classified into Type 2 by comparison with the results of the BSA-II ACIE (Example 5).

Conversely the sera of patients suffering from metastatic hepatic carcinoma showing Type 1 by BSA-II ACIE were classified into Type 3 by comparison with the results of the RCA-I ACIE (Example 3).

Example 12

Final differential diagnosis of cancer by combination of BSA-II and LCH:

10

The cases of obscure differentiation in Example 5, that is, sera of patients suffering from metastatic hepatic carcinoma showing Type 1 by BSA-II ACIE were classified into Type IVb by comparison with the results of the LCH ACIE.

Thus, it is shown that by comparing the results of individual ACIE's using selected pairs of lectins, differential diagnosis can confidently be made as between primary and metastatic hepatic carcinoma and between oophoroma and metastatic hepatic carcinoma.

**Claims**

1. A method for the differential diagnosis of hepatic carcinoma in a patient, which comprises subjecting a sample of body fluids from the patient to a lectin-based affinity cross-linked immuno electrophoresis (ACIE) assay to determine the specific binding pattern of $\alpha$-fetoprotein(AFP) fractions in the sample, characterised in that the method comprises establishing a differential diagnosis as between primary hepatic carcinoma on the one hand and metastatic hepatic carcinoma on the other by the steps of carrying out a first lectin-based ACIE on a first sample of said body fluid with a first lectin selected from the following lectin pairs:

Con A : LCH
Con A : PHA-E
RCA -I : LCH
RCA -I : BSA-II
or
BSA -II : LCH

to establish a first AFP specific fraction binding pattern for that first lectin, carrying out a second lectin-based ACIE assay on a second sample of the same body fluid but using the second member of said selected lectin pair, thereby to establish a second AFP specific fraction binding pattern for that second lectin, and using the patterns thus obtained to make the said differential diagnosis.

2. A method for the differential diagnosis of hepatic carcinoma and oophoroma in a patient, which comprises subjecting a sample of body fluids from the patient to a lectin-based affinity cross-linked immuno electrophoresis (ACIE) assay to determine the specific binding pattern of $\alpha$-fetoprotein (AFP) fractions in the sample, characterised in that the method comprises establishing a differential diagnosis as between oophoroma on the one hand and metastatic hepatic carcinoma on the other by the steps of carrying out a first lectin-based ACIE on a first sample of said body fluid with a first lectin selected from the following lectin pairs:

PHA - E : RCA-I
PHA - E : BSA-II

to establish a first AFP specific fraction binding pattern for that first lectin, carrying out a second lectin-based ACIE assay on a second sample of the same body fluid but using the second member of said selected lectin pair, thereby to establish a second AFP specific fraction binding pattern for that second lectin, and using the patterns thus obtained to make the said differential diagnosis.

3. A method according to Claim 1 or 2, wherein the samples used are blood serum or plasma samples.

4. A method according to Claim 1, 2 or 3 wherein following the ACIE the AFP specific binding patterns are developed using POD-labelled protein A, 4-methoxy-1-naphthol and hydrogen peroxide as the colouring system.

**Patentansprüche**

1. Verfahren zur Differentialdiagnose eines Leberkarzinoms in einem Patienten, umfassend die Durchführung eines Affinitäts-Vernetzung-Immunelektrophoresetests (ACIE) auf Lectinbasis an einer Probe von Körperflüssigkeiten des Patienten zur Bestimmung des spezifischen Bindungsmusters von $\alpha$-Fetoprotein (AFP)-Fraktionen in der Probe, **dadurch gekennzeichnet**, daß das Verfahren die Erstellung einer Differentialdiagnose zwischen einem primären Leberkarzinom einerseits und einem metastasischen Leberkarzinom andererseits durch folgende Schritte umfaßt: Durchführung einer ersten ACIE auf Lectin-Basis an einer ersten Probe der Körperflüssigkeit mit einem ersten Lectin, ausgewählt aus den folgenden Lectinpaaren:

Con A : LCH
Con A : PHA-E
RCA -I : LCH
RCA -I : BSA-II
oder
BSA -II : LCH

um ein erstes Bindungsmuster AFP-spezifischer Fraktionen für das erste Lectin zu erstellen, Durchführung eines zweiten ACIE-Tests auf Lectinbasis an einer zweiten Probe der gleichen Körperflüssigkeit, jedoch unter Verwendung des zweiten Bestandteils des ausgewählten Lectinpaares, wodurch ein zweites Bindungsmuster AFP-spezifischer Fraktionen für das zweite Lectin erstellt wird, und Verwendung der so erhaltenen Muster zur Erstellung der Differentialdiagnose.

**2.** Verfahren zur Differentialdiagnose eines Leberkarzinoms und Oophoroms in einem Patienten, umfassend die Durchführung eines Affinitäts-Vernetzung-Immunelektrophoresetests (ACIE) auf Lectinbasis an einer Probe von Körperflüssigkeiten des Patienten zur Bestimmung des spezifischen Bindungsmusters von $\alpha$-Fetoprotein (AFP)-Fraktionen in der Probe, **dadurch gekennzeichnet**, daß das Verfahren die Erstellung einer Differentialdiagnose zwischen Oophorom einerseits und einem metastatischen Leberkarzinom andererseits durch folgende Schritte umfaßt: Durchführung einer ersten ACIE auf Lectinbasis an einer ersten Probe der Körperflüssigkeit mit einem ersten Lectin ausgewählt aus den folgenden Lectinpaaren:

PHA - E : RCA-I
PHA - E : BSA-II

um ein erstes Bindungsmuster AFP-spezifischer Fraktionen für das erste Lectin zu erstellen, Durchführung eines zweiten ACIE-Tests auf Lectinbasis an einer zweiten Probe der gleichen Körperflüssigkeit, jedoch unter Verwendung des zweiten Bestandteils des ausgewählten Lectinpaares, wodurch ein zweites Bindungsmuster AFP-spezifischer Fraktionen für das zweite Lectin erstellt wird, und Verwendung der so erhaltenen Muster zur Erstellung der Differentialdiagnose.

**3.** Verfahren nach Anspruch 1 oder 2, wobei die verwendeten Proben Blutserum- oder Plasmaproben sind.

**4.** Verfahren nach Anspruch 1, 2 oder 3, wobei nach der Durchführung der ACIE die AFP-spezifischen Bindungsmuster unter Verwendung von POD-markiertem Protein A, 4-Methoxy-1-naphthol und Wasserstoffperoxid als Färbesystem entwickelt werden.

**Revendications**

**1.** Méthode de diagnostic différentiel de cancers hépatiques chez un patient, qui comprend le fait de soumettre un échantillon de liquide corporel du patient à un test d'immunoélectrophorèse croisée d'affinité (ACIE) réalisé à l'aide de lectines, dans le but de déterminer le diagramme particulier de liaison des fractions d'$\alpha$-foetoprotéine (AFP) contenues dans l'échantillon, caractérisée en ce que cette méthode comporte l'établissement d'un diagnostic différentiel entre d'une part un cancer primitif du foie et d'autre part un cancer métastatique du foie, par les étapes consistant à effectuer, sur un premier échantillon dudit liquide corporel, une première ACIE à l'aide d'une première lectine choisie dans l'une des paires de lectines suivantes :
Con A : LCH
Con A : PHA-E
RCA-I : LCH
RCA-I : BSA-II
BAS-II : LCH
pour établir un premier diagramme spécifique de liaison des fractions d'AFP pour cette première lectine, à effectuer un second test d'ACEI sur un second échantillon du même liquide corporel, mais en utilisant le second élément de ladite paire choisie de lectines, pour établir ainsi un second diagramme spécifique de liaison des fractions d'AFP pour cette seconde lectine, et à utiliser les diagrammes obtenus pour effectuer ledit diagnostic différentiel.

2. Méthode de diagnostic différentiel de cancer hépatique et d'oophorome chez un patient, qui comprend le fait de soumettre un échantillon de liquide corporel du patient à un test d'immunoélectrophorèse croisée d'affinité (ACIE), à l'aide de lectines, pour déterminer le diagramme spécifique de liaison des fractions d'$\alpha$-foetoprotéine (AFP) contenues dans l'échantillon, caractérisée en ce que la méthode comporte l'établissement d'un diagnostic différentiel entre un oophorome d'une part et un cancer hépatique métastatique d'autre part, par les étapes consistant à effectuer une première ACIE sur un premier échantillon dudit liquide corporel, avec une première lectine choisie dans l'une des paires suivantes de lectines :

PHA-E : RCA-I

PHA-E : BSA-II

de façon à établir un premier diagramme spécifique de liaison des fractions d'AFP pour cette première lectine, à effectuer un second test d'ACIE sur un second échantillon du même liquide corporel, mais en utilisant le second élément de ladite paire choisie de lectines, de façon à établir un second diagramme spécifique de liaison des fractions d'AFP pour cette seconde lectine, et à utiliser les diagrammes ainsi obtenus pour effectuer ledit diagnostic différentiel.

3. Méthode selon la revendication 1 ou 2, dans laquelle les échantillons utilisés sont des échantillons de sérum ou de plasma sanguin.

4. Méthode selon la revendication 1, 2 ou 3, dans laquelle, à la suite de l'ACIE, les diagrammes spécifiques de liaison d'AFP sont développés à l'aide de protéine A marquée au POD, de 4-méthoxy-1-naphtol et de peroxyde d'hydrogène, en guise de système chromogène.